(19) Europäisches Patentamt
European Patent Office
Office européen des brevets

(11) Publication number: **0 232 736 A1**

(12) **EUROPEAN PATENT APPLICATION**

(21) Application number: 87100423.0

(22) Date of filing: 15.01.87

(51) Int. Cl.[4]: C07D 493/10 , //G01N33/78

(30) Priority: 06.02.86 US 827841

(43) Date of publication of application: 19.08.87 Bulletin 87/34

(84) Designated Contracting States: AT BE CH DE FR GB IT LI NL

(71) Applicant: ABBOTT LABORATORIES
14th Street and Sheridan Road North St
North Chicago Illinois 60064(US)

(72) Inventor: Fino, James Robert
231 Southwick Court
Vernon Hills Illinois 60061(US)
Inventor: Shipchandler, Mohammed Tyebji
640 Burdick Street
Libertyville Illinois 60048(US)

(74) Representative: Modiano, Guido et al
MODIANO, JOSIF, PISANTY & STAUB
Modiano & Associati Via Meravigli, 16
I-20123 Milan(IT)

(54) **Preparation of 4'-aminomethylfluorescein derivatives for use in fluorescence polarization immunoassays.**

(57) The present invention is directed to an improved method for the preparation of 4'-aminomethylfluorescein derivatives for use in fluorescence polarization immunoassays.

EP 0 232 736 A1

# PREPARATION OF 4'-AMINOMETHYLFLUORESCEIN DERIVATIVES FOR USE IN FLUORESCENCE POLARIZATION IMMUNOASSAYS

## REFERENCE TO RELATED APPLICATION

This application is a continuation-in-part of applicants' earlier U.S. application, Serial No. 732,461, filed May 8, 1985.

## BACKGROUND OF THE INVENTION

### Technical Field

The present invention relates to an improved method for the preparation of 4'-aminomethylfluorescein derivatives which may be employed as reagents in fluorescence polarization immunoassays.

### Background Art

Competitive binding immunoassays provide a means for determining the concentration of ligands in biological fluids such as serum, plasma, spinal and amniotic fluids, and urine.

The ligand to be measured competes with a labeled reagent, or "ligand-analog" or "tracer", for a limited number of receptor binding sites on antibodies specific to the ligand and ligand-analog. For purposes of this disclosure, the term "ligand-analog" refers to a compound possessing one or more determinant or epitopic sites capable of competing with the ligand for the binding sites of a receptor, whether in conjugation with a fluorescein derivative to form a tracer or in its unconjugated form. The concentration of ligand in the sample determines the amount of ligand analog which binds to the antibody: the amount of ligand analog that will bind is inversely proportional to the concentration of ligand in the sample, because the ligand and the tracer each bind to the antibody in proportion to their respective concentrations.

Fluorescence polarization provides a quantitative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is adsorbed and emitted. In contrast, when an unbound tracer is excited by plane polarized light, its rotation is much faster than that of the corresponding tracer-antibody conjugate. As a result, the light emitted from the unbound tracer molecules is depolarized.

The use of 4'-aminomethylfluorescein derivatives as tracers in fluorescence polarization immunoassays is known in the art, having been disclosed in U.S. Patent No. 4,510,251 to Kirkemo and Shipchandler. The method disclosed therein for the preparation of 4'-aminomethylfluorescein is not without significant drawbacks, however, as it has subsequently been learned by applicants that alkylation occurs as an unavoidable side reaction, thereby diminishing the yield of 4'-aminomethylfluorescein. The present invention provides a method whereby 4'-aminomethylfluorescein can be prepared without concomitant alkylation and thus with greatly improved yield.

### Summary of the Invention

The invention relates to a new and improved method for the preparation of 4'-aminomethylfluorescein compounds having the following structural formula:

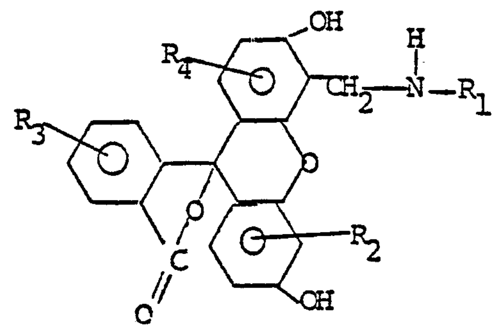

and acid addition salts thereof; wherein $R_1$ is hydrogen or a ligand-analog having at least one common epitope with a ligand so as to be specifically recognizable by a common antibody; and $R_2$, $R_3$ and $R_4$ each may be hydrogen, alkyl, halo, amino or carboxyl.

The novel process whereby these compounds may be prepared comprises reacting an acetamidomethylfluorescein or 4′-haloacetamidomethylfluorescein derivative having the following structural formula:

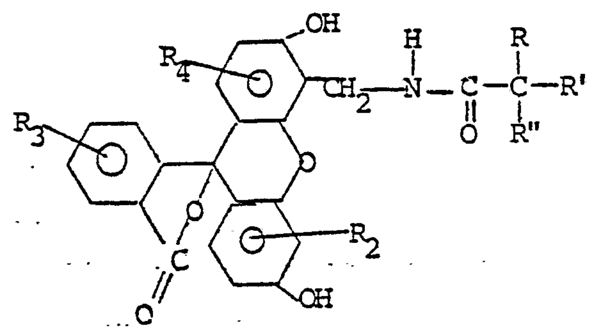

wherein R, R′ and R″ each may be hydrogen or halo; and $R_2$, $R_3$ and $R_4$ each may be hydrogen, alkyl, halo, amino or carboxyl; in the presence of a nonhydroxylic ether and a strong acid and recovering the 4′-aminomethylfluorescein thus produced.

## Detailed Description of the Invention

The present invention involves the use of fluorescein and derivatives of fluorescein and, more specifically, utilization of their ability to emit fluorescence. Fluorescein exists in either of two tautomeric forms depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. As used herein, the term "fluorescein", either as an individual compound or as a component of a larger compound, is meant to include both the open and closed forms, if they exist for a particular molecule, except in the context of fluorescence. An open form is necessary for the fluorescence to occur.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes rotation corresponding to the polarization observed. When a ligand competes with a tracer for antibody sites, then the observed polarization of fluorescence of the resulting mixture of free tracer and tracer-antibody complex assumes a value intermediate between that of the tracer and that of the tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be interpolated from a standard cure prepared in this manner.

The 4'-aminomethylfluorescein compounds prepared according to the teachings of the present invention generally exist in an equilibrium between their protonated and ionized states, and in the ionized state are effective in fluorescence polarization immunoassays. For convenience, the compounds are structurally represented herein in their protonated form. In their ionized state, the compounds exist in the form of biologically acceptable acid addition salts. As used herein, the term "biologically acceptable salts" refers to salts such as sodium, potassium, and ammonium which will enable the compounds to exist in their ionized state when employed in fluorescence polarization immunoassays. Generally, the compounds of the present invention exist in solution as salts, the specific salt resulting from the buffer employed. For example, in the presence of a sodium phosphate buffer, the compounds will generally exist in their ionized state as sodium salts.

Various 4'-aminomethylfluorescein derivatives useful as tracers can be prepared in accordance with the improved method of the present invention. Essentially, fluorescein is treated with acetamidomethanol or a 4'-haloacetamidomethanol in the presence of acid to yield an acetamidomethyl or 4'-haloacetamidomethyl derivative, which is then hydrolyzed in the presence of acid and a nonhydroxylic ether to yield an acid salt of a 4'-aminomethylfluorescein. No undesired alkylated side products are produced by this method. The 4'-aminomethylfluorescein so produced is treated with an activated ester of the ligand-analog of choice in the presence of a suitable solvent and a suitable base sufficient to neutralize the acid salt to yield a 4'-aminomethylfluorescein tracer.

The nonhydroxylic ether that is used will preferably have a boiling point in the range of 80-200°C and, more preferably, in the range of 130-200°C. Examples of preferred ethers include diethylene glycol dimethyl ether and ethylene glycol dimethyl ether.

The following illustrative, nonlimiting examples will serve to further demonstrate to those skilled in the art the manner in which 4'-aminomethylfluorescein compounds may be prepared in accordance with the invention.

## Example I

This example illustrates the preparation of 4'-chloroacetamidomethylfluorescein, which serves as an intermediate in the preparation of 4'-aminomethylfluorescein.

To 10.0g (30.1 mmole) of fluorescein dissolved in 100 ml of concentrated sulfuric acid was added 3.7 g (30.1 mmole) of N-hydroxymethylchloroacetamide as a solid, along with 100 ml of additional sulfuric acid. The reaction was stirred for 20 hours under an argon atmosphere protected from light and then poured over 1 liter of ice. The yellow precipitate was washed several times with ice-cold water and then dried under reduced pressure. The product was purified using column chromatography (7x50 cm column, 700 g silica gel). Elution was with 5% methanol in methylene chloride, followed by 10% methanol in methylene chloride. Fractions were checked by thin-layer chromatography (silica gel, 10% methanol in methylene chloride) after which the appropriate fractions were combined and the solvent was removed under reduced pressure. The yield was 4.9 g (37%) of chloroacetamidomethylfluorescein as an orange solid.

## Example II

This example illustrates the preparation of 4'-aminomethylfluorescein.

Chloroacetamidomethylfluorescein (25.0 g), prepared according to Example I, was refluxed in diethylene glycol dimethyl ether (110 ml) and concentrated hydrochloric acid (10 ml) for 20 hours under a nitrogen atmosphere. The solvent was removed under reduced pressure and the residue was purified using a Waters Prep LC/System 500 A liquid chromatograph equipped with two Prep PAK C18 columns. Elution at 100 ml/minute was with water: methanol: acetic acid (50:50:0.4). The residue was dissolved in methanol (40 ml) and four injections were made into the preparative system. Fractions were checked by thin-layer

chromatography (Whatman C18 plates, 40:60:0.4, 500 mM ammonium acetate:methanol:acetic acid), after which the appropriate fractions were combined. The solvent was removed under reduced pressure and the residual acetic acid was removed by coevaporation with methanol:toluene (1:1,3 x 100 ml). The yield was 6.8 g (31%) of 4'-aminomethylfluorescein hydrochloride as a dark orange solid.

It will be understood that various changes, modifications and equivalents can be made in the details of procedure, formulation and use without departing from the spirit and scope of the invention.

## Claims

1. A process for the preparation of 4'-aminomethylfluorescein compounds, and the acid addition salts thereof, comprising reacting an acetamidomethylfluorescein or 4'-haloacetamidomethylfluorescein in the presence of a nonhydroxylic ether and a strong acid and recovering the 4'-aminomethylfluorescein compound thus produced.

2. A process as defined in claim 1 wherein the nonhydroxylic ether is diethylene glycol dimethyl ether.

3. A process as defined in claim 1 wherein the strong acid is concentrated hydrochloric acid.

4. A process as defined in claim 1 wherein the 4'-haloacetamidomethylfluorescein is 4'-chloroacetamidomethylfluorescein.

European Patent Office

# EUROPEAN SEARCH REPORT

Application number

EP 87100423.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP - A1 - 0 110 186 (ABBOTT LABORATORIES)<br>* Claims 1,11,13 *<br>-- | 1,3,4 | C 07 D 493/10<br>//G 01 N 33/78 |
| A | CHEMICAL ABSTRACTS, vol. 69, no. 1, July 1, 1968, Columbus, Ohio, USA<br>DE LOPIDANA, MONT SERRAT GRAS G. "Metallfluorescent indicators" page 750, column 2, abstract no. 8 009e<br>& Acta Client. Compostelana 1966, 3(4), 173-80<br>-- | 1 | |
| D,A,P | EP - A1 - 0 200 960 (ABBOTT LABORATORIES)<br>* Abstract; fig. 10 *<br>---- | 1 | TECHNICAL FIELDS SEARCHED (Int. Cl.4)<br>C 07 D 493/00 |

The present search report has been drawn up for all claims

| Place of search | Date of completion of the search | Examiner |
|---|---|---|
| VIENNA | 12-05-1987 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82